(19) **Europäisches Patentamt**

**European Patent Office**

**Office européen des brevets**

(11) **EP 0 883 575 B1**

(12) **EUROPÄISCHE PATENTSCHRIFT**

(45) Veröffentlichungstag und Bekanntmachung des Hinweises auf die Patenterteilung:
**07.06.2000 Patentblatt 2000/23**

(51) Int Cl.$^7$: **C02F 3/34**, C02F 3/28, C02F 1/62

(21) Anmeldenummer: **97905008.5**

(22) Anmeldetag: **10.02.1997**

(86) Internationale Anmeldenummer:
**PCT/EP97/00597**

(87) Internationale Veröffentlichungsnummer:
**WO 97/29054 (14.08.1997 Gazette 1997/35)**

(54) **VERFAHREN ZUM BEHANDELN EINER SAUREN LÖSUNG**

METHOD OF TREATING AN ACID SOLUTION

PROCEDE PERMETTANT DE TRAITER UNE SOLUTION ACIDE

(84) Benannte Vertragsstaaten:
**AT BE CH DE DK ES FI FR GB GR IE IT LI LU MC NL PT SE**

(30) Priorität: **09.02.1996 DE 19604689**

(43) Veröffentlichungstag der Anmeldung:
**16.12.1998 Patentblatt 1998/51**

(73) Patentinhaber: **FACHHOCHSCHULE KONSTANZ D-78462 Konstanz (DE)**

(72) Erfinder:
• **Kreikenbohm, Rainer, Dr.**
**78224 Singen (DE)**
• **Gümpel, Paul, Prof. Dr.-Ing.**
**78351 Bodman-Ludwigshafen (DE)**

(74) Vertreter: **Behrmann, Niels, Dipl.-Ing. et al**
**Hiebsch Peege Behrmann,**
**Patentanwälte,**
**Heinrich-Weber-Platz 1**
**78224 Singen (DE)**

(56) Entgegenhaltungen:
**EP-A- 0 436 254       US-A- 4 789 478**

• **PATENT ABSTRACTS OF JAPAN vol. 006, no. 078 (C-102), 15.Mai 1982 & JP 57 012893 A (MITSUBISHI HEAVY IND LTD), 22.Januar 1982,**
• **DATABASE WPI Section Ch, Week 9520 Derwent Publications Ltd., London, GB; Class D15, AN 95-153512 XP002033446 & SU 1 838 598 A (SHUGINA G A ) , 30.August 1993**

# EP 0 883 575 B1

## Beschreibung

Die vorliegende Erfindung betrifft ein Verfahren zum Behandeln einer sauren Lösung. Insbesondere betrifft die vorliegende Erfindung ein Verfahren zum Behandeln von saurem Beizabwasser, wie es in der metallverarbeitenden Industrie anfällt.

Es wird angenommen, daß in der Bundesrepublik Deutschland mehr als 20 000 Betriebe Metalloberflächen naß behandeln, wobei eine solche Oberflächenbehandlung von Metall in Form von Galvanisieren, Beizen, chemischem und elektrolytischem Abtragen od. dgl. erfolgt. Durch jedes dieser Fertigungsverfahren entstehen Abwässer, denen ein niedriger pH-Wert -- bedingt durch die eingesetzten Säuren -- sowie eine hohe Eisen- bzw. Schwermetallkonzentration durch die verschiedenen Legierungsbestandteile -- Nickel, Chrom, Molybdän, Vanadium od. dgl. -- gemeinsam sind. Ferner führt der Einsatz von Salpetersäure zu einer hohen Nitratkonzentration im Abwasser, und üblicherweise wird ebenfalls eine hohe Konzentration an Fluoriden festgestellt.

Bisherige Verfahren zur Behandlung von derartigen Abwässern mit der Absicht, den Schwermetall-, Nitrat- und/oder Fluoridgehalt unter die gesetzlich festgelegten Grenzwerte abzusenken, basieren auf dem Zusatz verschiedener Chemikalien, mit denen die einzelnen Schadstoffe entfernt werden. Beispielsweise wird Beizabwasser der eingangs beschriebenen Art mittels Natriumdithionit oder Natriumpyrosulfit behandelt, um sechswertige Chromverbindungen zu dreiwertigen zu reduzieren. Daraufhin werden die Schwermetalle unter Einsatz von Natronlauge bzw. Natriumsulfid in schwerlösliche Metallhydroxide bzw. Metallsulfide überführt, und verbliebenes Fluorid wird mit Kalkmilch gefällt.

Nachteilig an dieser bekannten Art der Behandlung von Abwasser ist -- neben dem beträchtlichen Verbrauch an zuzusetzenden Chemikalien -- die Erzeugung von teilweise schwer sedimentierbaren Schlämmen sowie die Aufsalzung des Abwassers.

Aufgabe der vorliegenden Erfindung ist es daher, ein Verfahren zum Behandeln einer sauren, Eisen und/oder ein Schwermetall sowie ein Nitrat enthaltenden Lösung zu schaffen, das gegenüber dem beschriebenen bekannten Verfahren vereinfacht und im Hinblick auf entstehende Reststoffe umweltverträglicher gestaltet ist, das zudem insbesondere auch eine Abwasserbehandlung mit besserem Wirkungsgrad und verbessertem Reinigungsergebnis ermöglicht.

Die Aufgabe wird durch das Verfahren nach dem Patentanspruch 1 gelöst. Vorteilhafte Weiterbildungen der Erfindung sind in den Unteransprüchen beschrieben.

So bewirkt das erfindungsgemäße Verfahren zum Behandeln einer sauren Lösung eine Schwermetallelimination mittels einer Kombination aus mikrobiellen, physikalisch-chemischen und biophysikalischen Prozessen, die nacheinander ablaufen. Dabei unterstützt die als Kohlenstoff- und Energiequelle dienende Substanz Prozesse der Denitrifikation und Sulfatreduktion, die mikrobiell ablaufen.

Gemäß einer bevorzugten Ausführungsform der Erfindung werden als Kohlenstoff- und Energiequelle Lösungsmittel -- bevorzugt in Form von Alkoholen, Estern, Ketonen und Glykolen oder Mischungen davon -- gezielt zugesetzt.

Beispiele von Alkoholen sind primäre, sekundäre und tertiäre Alkohole der allgemeinen Formel

$$R_1R_2R_3C\text{-OH},$$

wobei $R_1$, $R_2$ und $R_3$ die Bedeutung von Wasserstoff, Methyl, Ethyl, Propyl, 2-Propyl, Butyl, Isobutyl, Pentyl usw. haben können. Methanol ist besonders bevorzugt.

Beispiele von Estern sind Verbindungen der allgemeinen Formel

$$R_1\text{-CO-O-}R_2,$$

wobei $R_1$ und $R_2$ die Bedeutung von Methyl, Ethyl, Propyl, 2-Propyl, Butyl, Isobutyl, Pentyl usw. haben können. Ethylacetat ist besonders bevorzugt.

Beispiele von Ketonen sind Verbindungen der allgemeinen Formel

$$R_1R_2\,C = O,$$

wobei $R_1$ und $R_2$ die Bedeutung von Methyl, Ethyl, Propyl, 2-Propyl, Butyl, Isobutyl, Pentyl, Benzyl usw. haben können.

Beispiele von Glykolen sind Verbindungen der allgemeinen Formel

$$(R_1)_2 \, HC(OH)\text{-}(OH) \, CH(R_1)_2,$$

wobei $R_1$ die Bedeutung von Wasserstoff, Methyl, Ethyl, Propyl, 2-Propyl, Butyl, Isobutyl usw. sein können. Weitere Beispiele von Glykolen sind Diethylenglykol, Triethylenglykol, 1,3-Butylenglykol, Hexylenglykol, Polyethylenglykole oder Dipropylenglykol.

Damit die Denitrifikation und die Sulfatreduktion als Stoffwechselprozesse der Bakterien ablaufen können, muß ferner neben der Kohlenstoff- und Energiezufuhr Sulfat als Elektronenakzeptor zugegeben werden, und der pH-Wert darf einen Wert von etwa pH = 3,5 nicht unterschreiten.

Als Ergebnis der Zugabe der einzelnen Stoffe zu der zu behandelnden sauren Lösung in der erfindungsgemäßen Weise laufen die folgenden Prozesse ab:

a) Denitrifikation -- im wesentlichen mikrobielle Umsetzung des Nitrats zu $N_2$

b) Sulfatreduktion -- im wesentlichen mikrobielle Umsetzung von $SO_4^{2-}$ zu $HS^-$

c) Schwermetallausfällung, beispielsweise im Wege einer Metallsulfidfällung

d) Fluoridbiosorption -- beispielsweise im Wege der Anlagerung 2wertiger Kationen an die negativ geladenen Exopolysaccharide (EPS) der Bakterien, woran nachfolgend $F^-$ gebunden werden kann.

Am Ende dieses erfindungsgemäßen Verfahrens steht dann ein gereinigtes Abwasser sowie eine mit Schwermetallsulfiden und Fluorid beladene Biomasse, wobei eine weitere Aufarbeitung dann mittels -- bekannter -- metallurgischer Verfahren erfolgen kann.

Erfindungsgemäß erfolgen also die Prozesse der Denitrifikation und der Sulfatreduktion mikrobiell, und zusätzlich kommt es zu einer Biosorption von Kationen an die negativ geladene Außenmembran der Bakterien, die auf diese Weise Bindungsplätze für die negativ geladenen Fluoridionen zur Verfügung stellen.

Anhand der folgenden Beispielreaktionen wird das der Erfindung zugrundeliegende Lösungsprinzip verdeutlicht, wobei die dargestellten Reaktionen rein exemplarischen Charakter haben und -- je nach Zusammensetzung des zu behandelnden Abwassers und der erfindungsgemäß zugegebenen Substanzen -- in jeweils entsprechender Weise ablaufen würden.

In diesem Beispiel wird angenommen, daß die zu behandelnde, saure Lösung die Schwermetalle Chrom und Nickel enthält, sowie ferner Fluoridionen aus der Lösung zu entfernen sind. Als Kohlenstoff- und Energiequelle wurden die Lösungsmittel Methanol sowie Ethylacetat zugegeben. Ferner wurde eine Anreicherungskultur mit anaerobem Schlamm und Biomasse in erfindungsgemäßer Weise für die stoffwechselphysiologisch aktiven Bakterien in einem speziellen Bioreaktor (Festbettreaktor) unter Zufuhr von Mineralsalzen und Spurenelementen zugesetzt bzw. angesiedelt.

Es ergeben sich die nachfolgenden -- aus energetischen Gründen in der beschriebenen Reihenfolge ablaufenden -- Prozeßschritte, wobei durch die Zufuhr des sauren Abwassers ein pH-Wert = 3,5 nicht unterschritten werden darf, damit der mikrobielle Denitrifikationsprozeß einsetzen kann (durch die Zufuhr einer extrem sauren Lösung kann leicht eine zu große lokale Störung in einer nur langsam umgewälzten Reaktorflüssigkeit auftreten, die vorab besser abgepuffert werden sollte; falls dies nicht möglich ist, muß das zugepumpte Volumen entsprechend klein gewählt werden. Ein pH-Wert von pH = 5 sollte im Reaktor mittelfristig nicht unterschritten werden):

1. Die Umsetzung von Methanol (als Beispiel für ein Lösungsmittel) mit Nitrat durch die Bakterien:

$$5 \, CH_3OH + 6 \, NO_3^- + H^+ \rightarrow 5 HCO_3^- + 3 \, N_2 + 8 \, H_2O.$$

2. Durch den Nitratverbrauch erfolgt pH-Wert-Anhebung und Fällung von Chrom:

$$Cr^{3+} + 3 \, OH^- \rightarrow Cr(OH)_3.$$

(Die Fällung von Chrom kann auch über die Zufuhr von Lauge erreicht werden).

3. Die Sulfatreduktion setzt ein, am Beispiel der Umsetzung von Ethylacetat gezeigt:

$$2\ C_4H_8O_2 + 5\ SO_4^{2-} \rightarrow 8\ HCO_3^- + 5\ HS^- + 3\ H^+.$$

Die Sulfatreduktion gemäß Prozeßschritt 3. setzt sich aus zwei Teilschritten zusammen:

a) $\quad 2\ C_4H_8O_2 + SO_4^{2-} \rightarrow 4CH_3COO^- + HS^- + 3\ H^+$

b) $\quad CH_3COO^- + SO_4^{2-} \rightarrow 2\ HCO_3^- + HS^-$

Der Prozeß ist dann optimal eingestellt, wenn beide Umsetzungen gemäß der Teilschritte a) und b) ablaufen, so daß die maximale Sulfidkonzentration erreicht wird.

4. Einstellung des Sulfidgleichgewichts:

$$HS^- \leftrightarrow S^{2-} + H^+.$$

5. Fällung von Nickel mit $S^{2-}$-Ionen:

$$Ni^{2+} + S^{2-} \rightarrow NiS.$$

6. Anlagerung von zweiwertigen Kationen $Ca^{2+}$ an die negativ geladenen Exopolysaccharide (EPS) der Bakterien und nachfolgendes Binden von $F^-$:

$$EPS^- + Ca^{2+} \rightarrow EPSCa^+,$$

$$EPSCa^+ + F^- \rightarrow EPSCaF.$$

Als Ergebnis dieser Prozesse wurde Chrom als $Cr(OH)_3$ und Nickel als NiS ausgefällt, während die Biomasse mit dem Fluorid beladen ist. Diese Abfallstoffe stehen nun zur Weiterbehandlung bzw. Entsorgung mittels üblicher Verfahren bereit.

Das erfindungsgemäße Verfahren ermöglicht daher gegenüber einem herkömmlichen Verfahren zur Behandlung saurer Beizabfälle eine wesentlich effizientere und umweltschonendere Aufarbeitung der Abwässer, die insbesondere die oben beschriebenen Nachteile der herkömmlichen Verfahren weitgehend vermeidet.

Zur Umsetzung von organischen Substanzen mit Nitrat als Elektronenakzeptor im Zuge der Denitrifikation (vergleiche oben Reaktion in Prozeßschritt 1) sind eine Vielzahl von Bakterienarten aus verschiedenen Gattungen befähigt. Aufgrund der Vorgaben an die Kohlenstoff- und Energiequellen sind folgende Arten geeignet:

<u>Paracoccus</u> <u>denitrificans</u>, <u>Pseudomonas</u> <u>stutzeri</u>, <u>Bacillus licheniformis</u>, <u>Hyphomicrobium</u> sp. sowie <u>Thiobacillus denitrificans.</u>

Stellvertretend für jene anaeroben Bakterien, die die vorgegebenen Substrate (Lösungsmittelgemisch) im Wege einer Sulfatreduktion umsetzen können, seien die folgenden Arten genannt, die bisher auch im mikroskopischen Bild beobachtet werden konnten: <u>Desulfobotulus saprovorans</u>, <u>Desulfovibrio vulgaris</u>, <u>Desulfotomaculum orientis</u>, <u>Desulfobacter postgatei</u> und <u>Desulfococcus multivorans</u>. <u>Desulfobotulus saprovorans</u> und <u>Desulfovibrio vulgaris</u> oxidieren -- entsprechend der Teilreaktion a) des Prozeßschrittes 3 -- eine Vielzahl von Alkoholen, Estern und Glykolen mit Sulfat unvollständig zu Acetat und Sulfid. Acetat wird dann von <u>Desulfobacter postgatei,</u> aber auch <u>Desulfococcus multivorans</u> vollständig mineralisiert. Die letztgenannte Bakterienart ist außerdem in der Lage, die angebotenen Lösungsmittel vollständig zu oxidieren.

Methanol wird bevorzugt von <u>Desulfotomaculum orientis</u> mineralisiert, und zwar gemäß

$$4\ CH_3OH + 3\ SO_4^{2-} \rightarrow 4\ HCO_3^- + 3\ HS^- + H^+ + 4\ H_2O$$

(betreffend Prozeßschritt 3.)

An den mikrobiellen Prozessen sind nur mesophile Bakterien beteiligt, deren Temperaturoptima im Bereich zwischen etwa 33° C und etwa 38° C liegen. Da einerseits die Werte für die Löslichkeitsprodukte der Schwermetallsulfide bei niedrigen Temperaturen fallen, wodurch die Effizienz der Ausfällung steigt, und außerdem die Verluste an Schwefelwasserstoff aufgrund höherer Flüchtigkeit mit steigender Temperatur zunehmen, wird für den gesamten Reinigungsvorgang erfindungsgemäß ein Temperaturbereich von T = 28 ± 5°C eingehalten. Aus Kostengründen ist es möglich und vorteilhaft, den Prozeß bei Raumtemperatur ablaufen zu lassen.

Da es sich bei den zum Einsatz kommenden, sulfatreduzierenden Bakterien um anaerobe Mikroorganismen handelt, die ihren Stoffwechsel nur unter anoxischen Bedingungen ausführen, sollte das Redoxpotential in den Anaerobreaktoren -200 mV nicht überschreiten.

Erfindungsgemäß sollte der pH-Wert im Anaerobreaktor in einem Bereich zwischen etwa 6 und etwa 8,5 liegen, wobei das Optimum für den Gesamtprozeß bei pH = 7,5 ± 0,3 liegt. Für den Ablauf der Stoffwechselvorgänge der eingesetzten Bakterien sind einige der folgenden Spurenelemente unentbehrlich: Fe, Mn, Zn, Co, Ni, Cu, Se, W und Mo, wobei diese essentiellen Elemente in der Regel im zugeführten Abwasser vorhanden sind (für sulfatreduzierende Bakterien sind alle erwähnten Spurenelemente essentiell; im allgemeinen Fall sind jedoch für die Mikroorganismen nur Eisen, Zink und Mangan zum Wachstum notwendig). Vor Inbetriebnahme einer Anlage zur erfindungsgemäßen Schwermetallelimination sollte allerdings eine Spurenelementanalyse durchgeführt werden und die fehlenden Spurenelemente ggf. aus einer Vorratslösung in geeigneten Konzentrationen zugeführt werden.

Die typische Verweilzeit in einer Reaktoranlage zur erfindungsgemäßen Schwermetallelimination beträgt bevorzugt einen Tag. Allerdings ist es möglich, die Anlage so zu betreiben, daß eine im wesentlichen gleiche Reinigungsleistung auch bei einer Verminderung der Verweilzeit bis zu etwa 6 Stunden erreicht werden kann.

Prinzipiell können die in der Patentanmeldung einzelnen aufgeführten Verfahrensschritte (1.)-(6.) zu einer ein-oder mehrstufigen Prozeßführung zusammengefaßt werden. Je nach Abwasserzusammensetzung und Verfügbarkeit von Substraten für den Erhalt der bakteriellen Biomasse kann es sinnvoll sein, die biologischen Prozesse Denitrifikation (1.) und Sulfatreduktion (3.) in zwei Reaktoren getrennt ablaufen zu lassen, wobei die Zugabe von Sulfat erst in den zweiten Behälter erfolgt. Dadurch finden auch die Prozesse der Schwermetallausfällung getrennt statt, und zwar bildet sich der Niederschlag aus Chrom(III)-hydroxid (2.) hauptsächlich im ersten Reaktor, während im zweiten alle anderen Schwermetalle als Sulfide (5.) abgetrennt werden können.

Weitere Vorteile, Merkmale und Einzelheiten der Erfindung ergeben sich aus der nachfolgenden Versuchsbeschreibung sowie einer Vorrichtung zum Ausführen des erfindungsgemäßen Verfahrens anhand der Zeichnung. Diese zeigt in

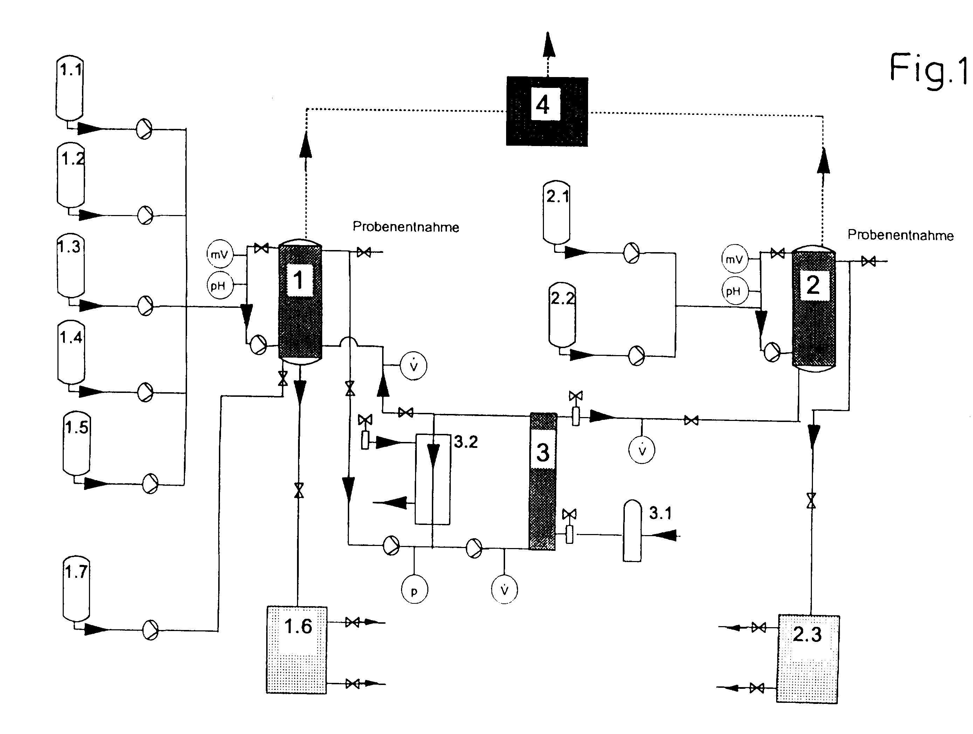

Fig. 1:    ein Fließschema einer Schwermetalleliminierungsanlage zur Durchführung des Verfahrens gemäß der vorliegenden Erfindung.

Bei einem Versuchsansatz wurde eine -- Nickel und Chrom sowie Fluorid enthaltene -- Abwasserprobe eines Beizmittels mit Anaerobschlamm, Sulfat, einer Mischung verschiedener Lösungsmittel sowie einer in Gegenwart von Blei gewachsenen Vorkultur inkubiert:

    27,5 ml Abwasser
    7,0 ml Anaerobschlamm mit Sulfat
    200 ml Lösungsmittelgemisch (3 M Ethylacetat,
    3 M Methanol, 3 M Ethylenglycol, 3 M 2-Butoxyethanol) 10,0 ml Vorkultur

auf 100 ml mit einem mineralischen, anaeroben Medium aufgefüllt.
    Inkubationstemperatur: 28° C
    Inkubationszeit: 5 Tage (Nickel, Chrom) bzw. 12 Tage (Nitrit und Nitrat).

Die Versuchsergebnisse sind der nachfolgenden Tabelle zu entnehmen, ferner die aus dem Abwasseranteil (27,5 %) berechneten Startwerte. Die Grenzwerte der vierten Spalte beruhen auf der Rahmen-Abwasser VwV Anhang 40.

Tabelle:

| Gemessene und berechnete Konzentrationen am Ausgangsmaterial und während des Versuchs sowie einzuhaltende Grenzwerte | | | |
|---|---|---|---|
| | Abwasserprobe | Startwert (berechnet) | Endwert (gemessen) | Grenzwert |
| Ni | 32 mg/l | 8,8 mg/l | 5 mg/l | 0,5 mg/l |
| Zn | 5 mg/l | 1,4 mg/l | n.b. | 2 mg/l |

Tabelle: (fortgesetzt)

| Gemessene und berechnete Konzentrationen am Ausgangsmaterial und während des Versuchs sowie einzuhaltende Grenzwerte | | | | |
|---|---|---|---|---|
| | Abwasserprobe | Startwert (berechnet) | Endwert (gemessen) | Grenzwert |
| Cr | 76 mg/l | 20,9 mg/l | 0,4 mg/l | 0,5 mg/l |
| F$^-$ | 1200 mg/l | 330 mg/l | 79 mg/l | 20 mg/l |
| NO$_3^-$ | 377 mg N/l | 104 mg N/l | < 2,8 mg N/l | -- |
| NO$_2^-$ | n.b. | n.b. | 0,28 mg N/l | 5 mg N/l |
| pH | 2,85 | 3,4* | 5,9 | -- |

n.b. = nicht bestimmt
* = gemessener pH-Wert

Im weiteren wird eine Vorrichtung zur Durchführung des erfindungsgemäßen Verfahrens anhand des Fließschemas einer Schwermetalleliminierungsanlage gemäß der Fig. 1 beschrieben.

Die Fig. 1 zeigt eine Darstellung einer Technikumsanlage zur Durchführung des erfindungsgemäßen Verfahrens zum Behandeln einer sauren Lösung.

Die dargestellte Anlage ist zur erfindungsgemäßen Behandlung insbesondere von Abwasser aus einer Verzinkerei vorgesehen, prinzipiell aber auch zum Durchführen des erfindungsgemäßen Verfahrens zum Behandeln anderer saurer Lösungen einsetzbar.

Die Technikumsanlage zur Schwermetallelimination in Fig. 1 zeigt:

1. Reaktor 1
1.1 Lauge
1.2 Säure
1.3 Sulfat
1.4 Kohlenstoff- und Energiequelle
1.5 Spurenelemente
1.6 Schlammabscheider
1.7 Abwasser

2. Reaktor 2
2.1 Lauge
2.2 Säure
2.3 Schlammabscheider

3. Ultrafiltration
3.1 Druckluft
3.2 Kühler

4. Eisenrasenstein

**Patentansprüche**

1. Verfahren zum Behandeln einer sauren, Eisen und/oder ein Schwermetall sowie ein Nitrat enthaltenden Lösung, mit den Schritten:

   - Zugeben einer als Kohlenstoff- und Energiequelle dienenden Substanz zu der Lösung,
   - Zugeben eines Sulfats zu der Lösung und Zusetzen von Bakterien, die für eine Adsorption (Biosorption) und eine Schwermetallfällung (Biopräzipitation) von in der Lösung vorhandenen oder entstehenden Kationen und/der Anionen eingerichtet sind.

2. Verfahren nach Anspruch 1, dadurch gekennzeichnet, daß die als Kohlenstoff- und Energiequelle dienende Substanz ein Lösungsmittel ist, das aus der Gruppe bestehend aus Alkoholen, Estern, Ketonen und Glykolen sowie Mischungen daraus ausgewählt wird.

3. Verfahren nach Anspruch 1 oder 2, gekennzeichnet durch den Schritt:

   - Einstellen des pH-Werts auf einen Wert von $\geq$ 3,5.

4. Verfahren nach einem der Ansprüche 1 bis 3, dadurch gekennzeichnet, daß als Sulfat Natriumsulfat zugegeben wird.

5. Verfahren nach einem der Ansprüche 1 bis 4, dadurch gekennzeichnet, daß das Verfahren bei einer Temperatur durchgeführt wird, die im Bereich zwischen etwa 23° C und etwa 33° C liegt.

6. verfahren nach einem der Ansprüche 1 bis 4, dadurch gekennzeichnet, daß das Verfahren bei Raumtemperatur durchgeführt wird.

7. Verfahren nach einem der Ansprüche 1 bis 6, dadurch gekennzeichnet, daß das Verfahren in einer Reaktoranlage durchgeführt wird, wobei eine Verweilzeit der Lösung, der Substanz, des Sulfats und der Bakterien in der Reaktoranlage einen Tag oder weniger beträgt.

8. Verfahren nach Anspruch 7, dadurch gekennzeichnet, daß die Reaktoranlage einen Anaerobreaktor aufweist und ein Redoxpotential in dem Anaerobreaktor auf einen Wert von -200mV oder kleiner eingestellt wird.

9. Verfahren nach einem der Ansprüche 1 bis 8, dadurch gekennzeichnet, daß die Bakterien aus der Gruppe ausgewählt werden, die aus den Arten Paracoccus denitrificans, Pseudomonas stutzeri, Bacillus licheniformis, Hyphomicrobium sp., Desulfobotulus saprovorans, Desulfovibrio vulgaris, Desulfotomaculum orientis, Desulfobacter postgatei sowie Desulfococcus multivorans besteht.

10. Verfahren nach einem der Ansprüche 1 bis 9, dadurch gekennzeichnet, daß der Schritt des Zugebens der als Kohlenstoff- und Energiequelle dienenden Substanz in einen ersten Reaktorbehälter und der Schritt des Zugebens des Sulfats in einen vom ersten Reaktorbehälter getrennten zweiten Reaktorbehälter erfolgt.

**Claims**

1. Method of treating an acid solution containing iron and/or a heavy metal and a nitrate, comprising the steps:

   - adding a substance serving as a carbon and energy source to the solution,
   - adding a sulphate to the solution and
   - adding bacteria intended for the adsorption (biosorption) and heavy-metal precipitation (bioprecipitation) of cations and/or anions present or produced in the solution.

2. Method according to claim 1, characterised in that the substance serving as a carbon and energy source is a solvent selected from the group consisting of alcohols, esters, ketones and glycols and mixtures thereof

3. Method according to claim 1 or claim 2, characterised by the step:

   - adjusting the pH value to a value of $\geq$ 3.5.

4. Method according to one of claims 1 to 3, characterised in that sodium sulphate is added as the sulphate.

5. Method according to one of claims 1 to 4, characterised in that the method is carried out at a temperature in the range between approximately 23°C and approximately 33°C.

6. Method according to one of claims 1 to 4, characterised in that the method is carried out at room temperature.

7. Method according to one of claims I to 6, characterised in that the method is carried out in a reactor plant, the dwell time of the solution, the substance, the sulphate and the bacteria in the reactor plant being one day or less.

8. Method according to claim 7, characterised in that the reactor plant has an anaerobic reactor and a redox potential in the anaerobic reactor is adjusted to a value of - 200 mV or less.

**9.** Method according to one of claims 1 to 8, characterised in that the bacteria are selected from the group consisting of the species Paracoccus denitrificans, Pseudomonas stutzen, Bacillus licheniformis, Hyphomicrobium sp., Desulfobotulus sparovorans, Desulfovibrio vulgaris, Desulfotomaculum orientis, Desulfobacter postgatei and Desulfococcus multivorans.

**10.** Method according to one of claims 1 to 9, characterised in that the step of adding the substance serving as a carbon and energy source is effected in a first reactor vessel and the step of adding the sulphate is effected in a second reactor vessel separate from the first reactor vessel.

**Revendications**

**1.** Un procédé de traitement d'une solution acide contenant du fer et/ou un métal lourd ainsi qu'un nitrate, comprenant les étapes suivantes :

- addition d'une substance servant de source de carbone et d'énergie à la solution.
- addition à la solution d'un sulfate et addition de bactéries qui sont disposées pour l'adsorption (biosorption) et précipitation des métaux lourds (bio-précipitation) des cations et/ou des anions présents ou formés dans la solution.

**2.** Un procédé selon la revendication 1, caractérisé en ce que la substance servant de source de carbone et d'énergie est un solvant choisi dans le groupe consistant en alcools, esters, cétones et glycols, ainsi que leurs mélanges.

**3.** Un procédé selon la revendication 1 ou 2, caractérisé par l'étape :

- ajustement de la valeur du pH à une valeur $\geq 3.5$.

**4.** Un procédé selon l'une quelconque des revendications 1 à 3, caractérisé en ce que, en tant que sulfate on ajoute du sulfate de sodium.

**5.** Un procédé selon l'une quelconque des revendications 1 à 4, caractérisé en ce que l'on met en oeuvre le procédé à une température qui se trouve dans la fourchette d'environ 23°C, à environ 33°C.

**6.** Un procédé selon l'une quelconque des revendications 1 à 4. caractérisé en ce que l'on met oeuvre le procédé à la température ambiante.

**7.** Un procédé selon l'une quelconque des revendications 1 à 6, caractérisé en ce que l'on met en oeuvre le procédé dans une installation de réacteurs dans lequel la durée de séjour de la solution de la substance du sulfate et des bactéries dans l'installation de réacteurs s'élève à un jour, ou moins.

**8.** Un procédé selon la revendication 7, caractérisé en ce que l'installation de réacteurs comporte un réacteur anaérobe et qu'on fixe le potentiel Redox dans le réacteur anaérobe à une valeur de - 200 mV, ou moins.

**9.** Un procédé selon l'une quelconque des revendications 1 à 8, caractérisé en ce que les bactéries sont choisies dans le groupe comprenant les espèces Paracoccus denitrificans, Pnseudomonas stutzeri, Bacillus licheniformis, llyphomicrobium sp., Desulfobotulus saprovorans, Desulfovibrio vulgaris, Desulfotomaculum orientis, Desulfobacter postgatei, ainsi que Desulfococcus multivorans.

**10.** Un procédé selon l'une quelconque des revendications 1 à 9, caractérisé en ce que l'étape d'addition de la substance servant de source de carbone et d'énergie est effectuée dans un premier réacteur et que l'étape d'addition du sulfate est effectuée dans un second réacteur séparé du premier réacteur.

Fig.1